# EUROPEAN PATENT APPLICATION

(11) **EP 3 203 230 A1**
(43) Date of publication of application: **09.08.2017**
(21) Application number: 17154432.3
(22) Date of filing: 02.02.2017
(51) Int. Cl.: G01N 33/497, A61B 5/097

(54) **BREATH COMPONENT MEASUREMENT APPARATUS**

(30) Priority: 04.02.2016 JP 2016019851
(71) Applicant: Hosiden Corporation, Yao-shi, Osaka 581-0071 (JP)
(72) Inventor: MOKUO, Shigeki, Yao-shi, Osaka 581-0071 (JP); KUWAKI, Junko, Yao-shi, Osaka 581-0071 (JP)
(74) Representative: MERH-IP Matias Erny Reichl Hoffmann Patentanwälte PartG mbB

(57) **Abstract**

A breath component measurement apparatus 1 includes a breath residence case 2. The breath residence case 2 includes a container 21 in the shape of a rectangular parallelepiped, an intake pipe 22 in the shape of a circular tube which is provided at the center of a front face of the container 21, a pump 23 which is formed over the whole right side surface of the container 21, a sensor storage unit 24 in the shape of a circular tube which is provided at the center of a bottom surface of the container 21, a gas sensor 25 which has the shape of a circular column and is stored in the sensor storage unit 24, a disk-shaped piezoelectric element 26 which is supported by a groove in a slot 27, the slot 27 that is formed on a left side surface of the container 21 and has the groove supporting the piezoelectric element 26, and an exhaust port 28 which is provided at a position hidden behind a back face of the piezoelectric element 26 of the left side surface of the container 21.

## Description

### [TECHNICAL FIELD]

The present invention relates to a breath component measurement apparatus for measuring a component contained in breath.

### [BACKGROUND ART]

An example of a breath component measurement apparatus of conventional semiconductor type is one disclosed in Japanese Patent Application Laid Open No. 2008-516198.

In the breath component measurement apparatus of conventional semiconductor type, breath flowing in through an intake port passes through a gas sensing unit and is exhausted through an exhaust port, and breath does not stay inside the apparatus for a long time. For this reason, the concentration of gas in the gas sensing unit may change depending on breath blowing intensity and the length of a blowing time to cause variations in measurement results.

### [SUMMARY OF THE INVENTION]

An object of the present invention is to provide a breath component measurement apparatus capable of reducing variations in measurement results.

A breath component measurement apparatus according to the present invention includes a breath residence case that causes breath to reside and a gas sensor that is provided inside the breath residence case.

### [EFFECTS OF THE INVENTION]

The breath component measurement apparatus according to the present invention allows a reduction in variations in measurement results.

### [BRIEF DESCRIPTION OF THE DRAWINGS]

Fig. 1 is a perspective view of a breath component measurement apparatus according to a first embodiment.
Fig. 2 is a right side view of the breath component measurement apparatus according to the first embodiment.
Fig. 3 is a right side view of a breath residence case according to the first embodiment.
Fig. 4 is a front-side perspective view of the breath residence case according to the first embodiment.
Fig. 5 is a back-side perspective view of the breath residence case according to the first embodiment.
Fig. 6 is a top-side perspective view showing an interior of the breath residence case according to the first embodiment when the breath residence case is cut.
Fig. 7 is a graph showing the relationship between the amount of influent breath and a difference in sensor output between before and after breath inflow.
Fig. 8 is a graph showing temporal change in sensor output before and after breath inflow and before and after forcible breath exhaust.
Fig. 9 is a block diagram showing a control system of the breath component measurement apparatus according to the first embodiment.
Fig. 10 is a flowchart showing the operation of a control unit of the breath component measurement apparatus according to the first embodiment.

### [DETAILED DESCRIPTION OF THE EMBODIMENTS]

An embodiment of the present invention will be described below in detail. Constituent units having the same functions are denoted by the same reference characters, and a repetitive description thereof will be omitted.

### FIRST EMBODIMENT

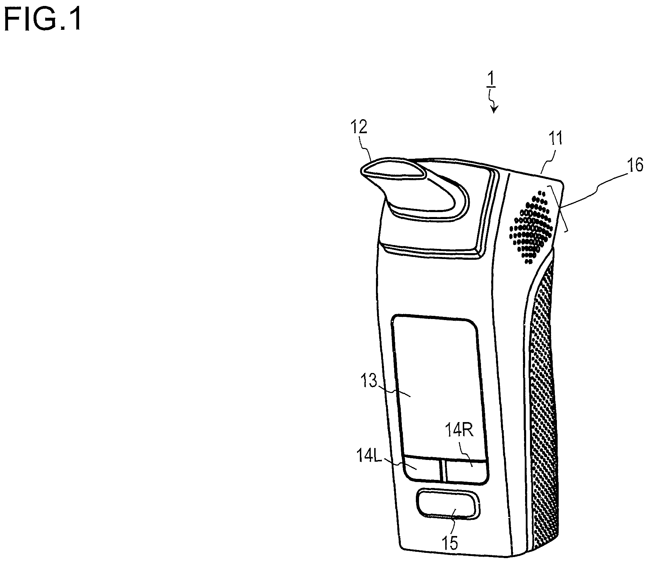

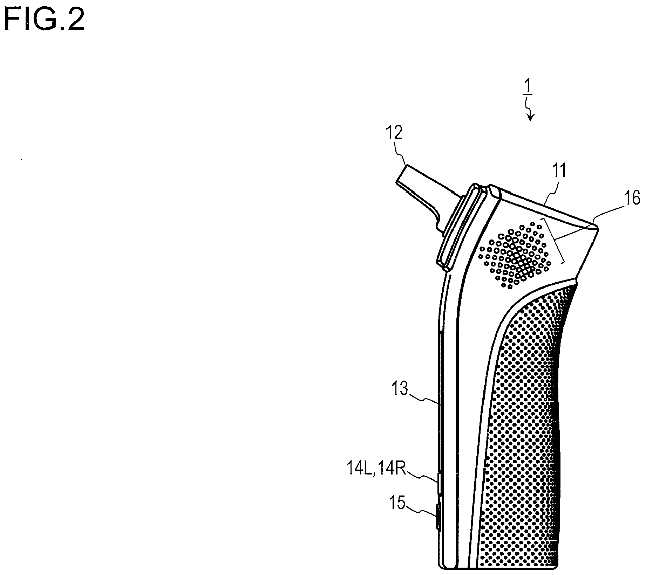

A breath component measurement apparatus according to a first embodiment will be described below with reference to Figs. 1 and 2. A breath component measurement apparatus 1 according to the present embodiment has the shape of a substantially quadrangular prism and includes a main body case 11 which is slightly curved toward the back and is more curved with an increase in vertical position, a tubular mouth piece 12 which is provided at an upper end of a front face of the main body case 11 and is wide and substantially elliptical in cross-section, a display unit 13 which is provided in the middle of the front face of the main body case 11, a left operation button 14L and a right operation button 14R which are provided on the front face of the main body case 11 and adjacent to each other at a lower portion of the display unit 13, a central operation button 15 which is provided at a lower portion of the front face of the main body case 11, and ventilation holes 16 which are provided at an upper portion of a right side surface and at an upper portion of a left side surface (not shown) of the main body case 11.

The display unit 13 is a liquid crystal screen or the like. In the present embodiment, each ventilation hole 16 is formed from circular holes arrayed in a plurality of rows and a plurality of columns. However, the ventilation hole 16 may be formed by arranging, for example, a plurality of oval holes or holes in any other shape.

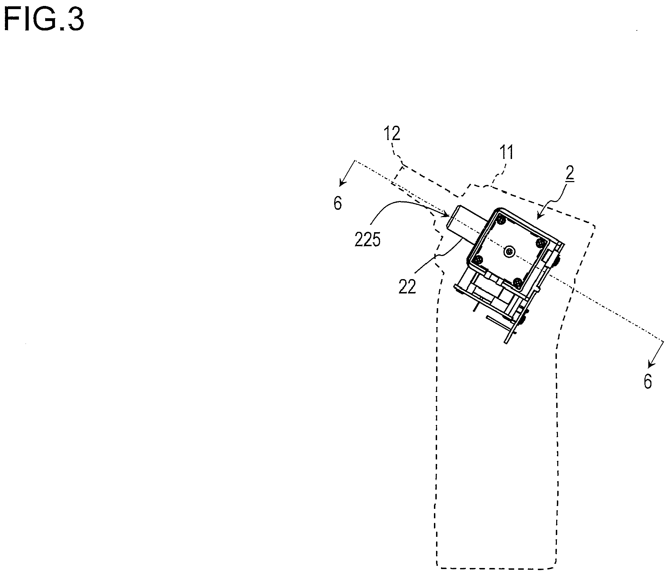

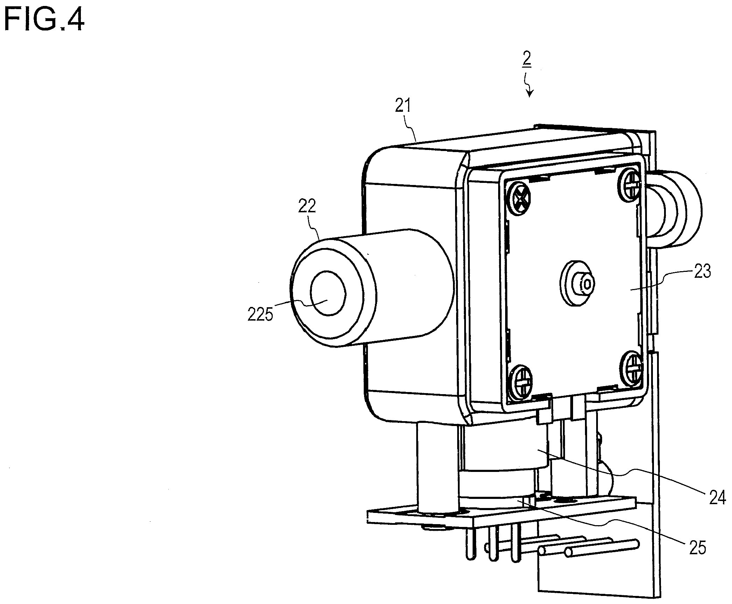

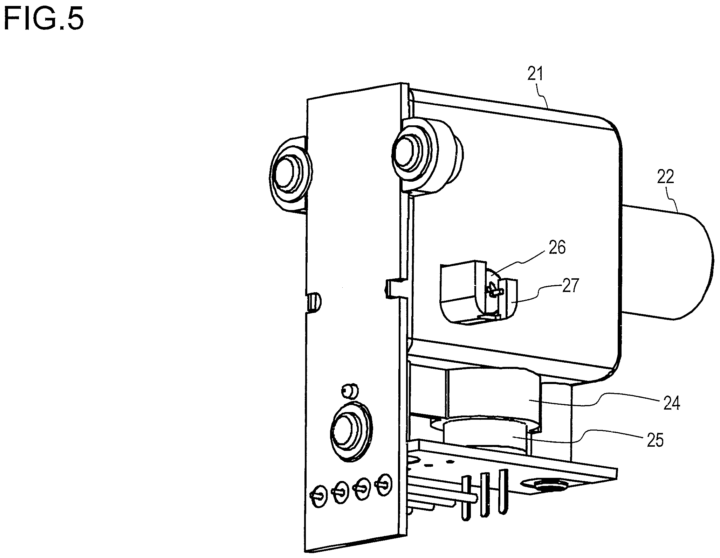

The breath residence case 2 stored inside the breath component measurement apparatus 1 will be described with reference to Figs. 3, 4, and 5. The breath residence case 2 is configured to include a container 21 in the shape of a rectangular parallelepiped, an intake pipe 22 in the shape of a circular tube which is provided at the center of a front face of the container 21, a pump 23 which is formed over the whole right side surface of the container 21, a sensor storage unit 24 in the shape of a circular tube which is provided at the center of a bottom surface of the container 21, a gas sensor 25 which has the shape of a circular column and is stored in the sensor storage unit 24, a disk-shaped piezoelectric element 26 which is supported by a groove in a slot 27 (to be described later), the slot 27 that is formed on a left side surface of the container 21 and has the groove supporting the piezoelectric element 26, and an exhaust port 28 which is provided at a position hidden behind a back face of the piezoelectric element 26 of the left side surface of the container 21. An opening portion of the intake pipe 22 is called an intake port 225. The piezoelectric element 26 and the left side surface of the container 21 are spaced apart, and a vacant space of fixed size is provided between the piezoelectric element 26 and the left side surface of the container 21.

Fig. 3 shows the positional relationship between the main body case 11 and the breath residence case 2 by a broken line. As shown in Fig. 3, breath blown through the mouth piece 12 is sent into the intake port 225 of the intake pipe 22.

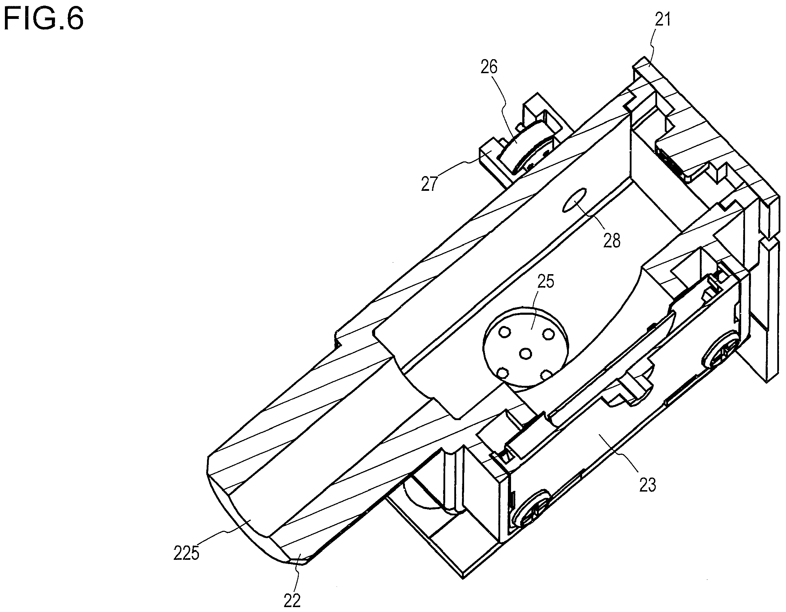

The internal structure of the container 21 will be described with reference to Fig. 6. Fig. 6 is a top-side perspective view showing an interior of the breath residence case 2 when the breath residence case 2 is cut along line 6-6 shown in Fig. 3. As shown in Fig. 6, an upper end portion of the gas sensor 25 is housed in the sensor storage unit 24 in the shape of a circular tube to seal the sensor storage unit 24, and breath is not exhausted or is unlikely to be exhausted from the sensor storage unit 24. An upper end face of the gas sensor 25 serves as a sensing unit which senses gas resident inside the container 21. The gas sensor 25 is connected to a control unit (for example, an IC chip or a microcomputer chip) (not shown) and transmits an electrical signal to the control unit. The control unit controls the display unit 13 in accordance with the electrical signal received from the gas sensor 25 to display a detection result. The control unit also executes various control operations, such as converting the electrical signal received from the gas sensor 25 into a user-editable data form and storing the electrical signal in a predetermined storage region. There is no particular limit to the type of gas to be sensed by the gas sensor 25. The gas may be, for example, alcohol, ketone, or acetone.

As shown in Fig. 6, breath which passes through the intake pipe 22 and flows into the container 21 is gradually exhausted through the exhaust port 28. If the exhaust port 28 is small with respect to the container 21, breath flowing into the container 21 is slow to be exhausted and is resident inside the container 21 for a longer time. On the other hand, if the exhaust port 28 is sufficiently large with respect to the container 21, breath flowing into the container 21 is promptly exhausted and is resident inside the container 21 for a shorter time.

Breath exhausted through the exhaust port 28 collides with the back face of the piezoelectric element 26, and the piezoelectric element 26 senses the exhaust and generates an electrical signal. The piezoelectric element 26 transmits the electrical signal to the control unit described earlier (not shown). Note that the breath colliding with the back face of the piezoelectric element 26 passes through the vacant space between the piezoelectric element 26 and the left side surface of the container 21 and is emitted mainly in an upward direction.

### <Relationship between Amount of Influent Breath and Volume of Container 21>

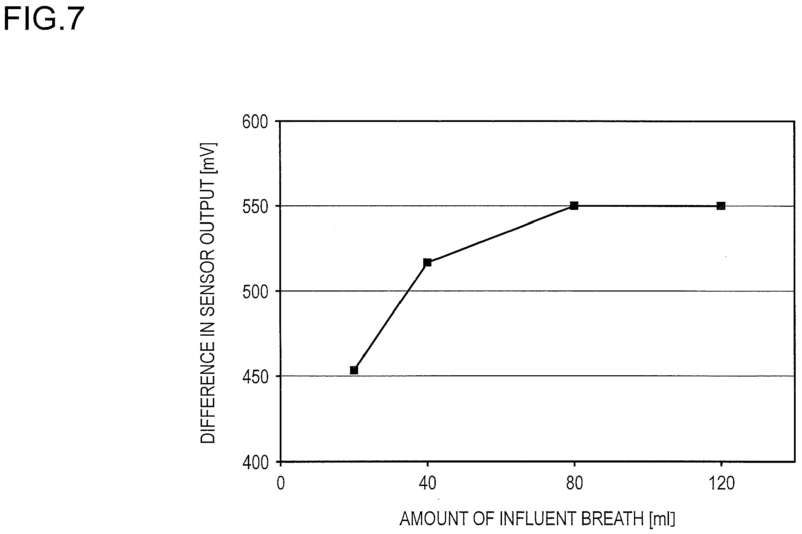

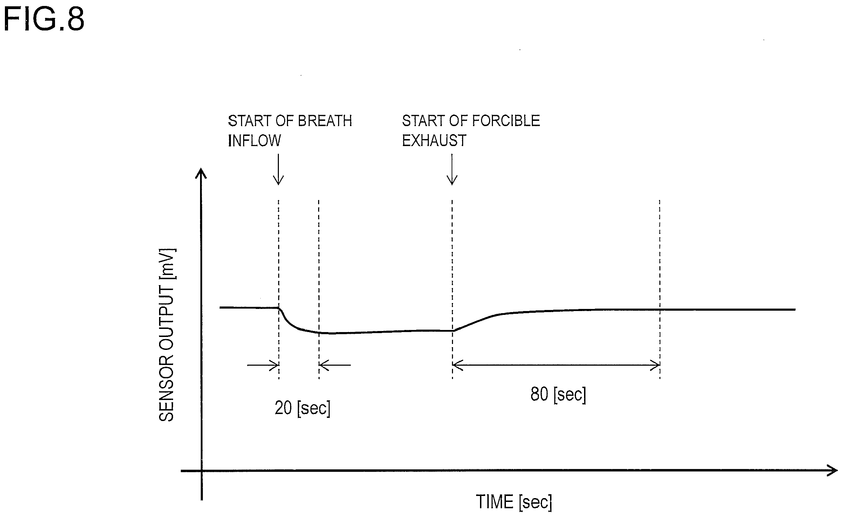

The relationship between the amount of influent breath and the volume of the container 21 will be described with reference to Figs. 7 and 8. Fig. 7 is a graph, on which a value of difference between a sensor output after each amount of breath flows into the container 21 and a sensor output under a clean atmosphere (an average value of a plurality of measurements) in a case where the volume of the container 21 is set to about 4.6 [ml] is plotted. The detailed measurement conditions are as follows. Sensor load resistance was 10 [kΩ], the bore of the intake port 225 was Φ4 [mm], and the bore of the exhaust port 28 was Φ2 [mm]. Breath when the stomach is empty before lunch was obtained as a sample of breath and put into a sample bag, and a fixed amount of breath was taken out of the sample bag and was injected into the intake port 225 using a syringe. Measurement was executed three times for each inflow amount.

For example, sensor outputs were 3700 to 3740 [mV] under a clean atmosphere. In contrast, sensor outputs were 3240 to 3300 [mV] if 10 [ml] of breath flowed in. Differences in sensor output were 440 to 470 [ml] (the average was 453.3 [ml]). Similarly, sensor outputs were 3180 to 3190 [mV] if 40 [ml] of breath flowed in. Differences in sensor output were 510 to 520 [ml] (the average was 516.7 [ml]). Sensor outputs were 3150 to 3160 [mV] if 80 [ml] of breath flowed in. Differences in sensor output were 550 [ml] and were constant regardless of the ordinal position of each measurement. Sensor outputs were 3150 to 3160 [mV] if 120 [ml] of breath flowed in, as in the case where 80 [ml] of breath flowed in. Differences in sensor output were 550 [ml] and were constant regardless of the ordinal position of each measurement.

It can be seen from Fig. 7 that a difference in sensor output comes closer to saturation with an increase in the amount of influent breath and reaches saturation. As described above, it is apparent that values of difference in sensor output tend to vary for different ordinal positions of measurements if the amount of influent breath is small and that values of difference tend to be more stable and vary less with an increase in the amount of influent breath.

As can be seen from the above-described measurement results, the amount (40 [ml]) of influent breath that is about 8.8 times the volume (about 4.6 [ml]) of the container 21 stabilizes measurement values for respective measurements and is a preferable condition. The amount (80 [ml]) of influent breath that is about 17.6 times the volume (about 4.6 [ml]) of the container 21 further stabilizes measurement values for respective measurements and brings the measurement values to sufficient saturation.

From the above-described measurement results, the container 21 or the breath residence case 2 is configured to have a capacity one-αth of a capacity which is set in advance as a standard breath blowing amount per blowing. The symbol α may be set in advance to be at least not less than 8.8, more preferably not less than 17.6.

### <Pump 23>

The pump 23 may be, for example, a piezoelectric micropump. The pump 23 may be a motor-powered pump or a fan motor. Fig. 8 shows a result of actually measuring change in sensor output before and after breath blowing and change in sensor output before and after forcible exhaust of breath by the pump 23. Fig. 8 represents a typical waveform of an actual measurement result in a case where the volume of the container 21 is set to about 4.6 [ml] and 80 [ml] of breath is blown in. As shown in Fig. 8, a sensor output becomes stable in about 20 [sec] after the start of breath inflow. The sensor output returns to an output equivalent to that under a clean atmosphere in about 80 [sec] after the start of forcible exhaust by the pump 23.

### <Piezoelectric Element 26>

The piezoelectric element 26 may be, for example, a microphone. The piezoelectric element 26 may be, for example, one of various pressure sensor elements.

### <Control System>

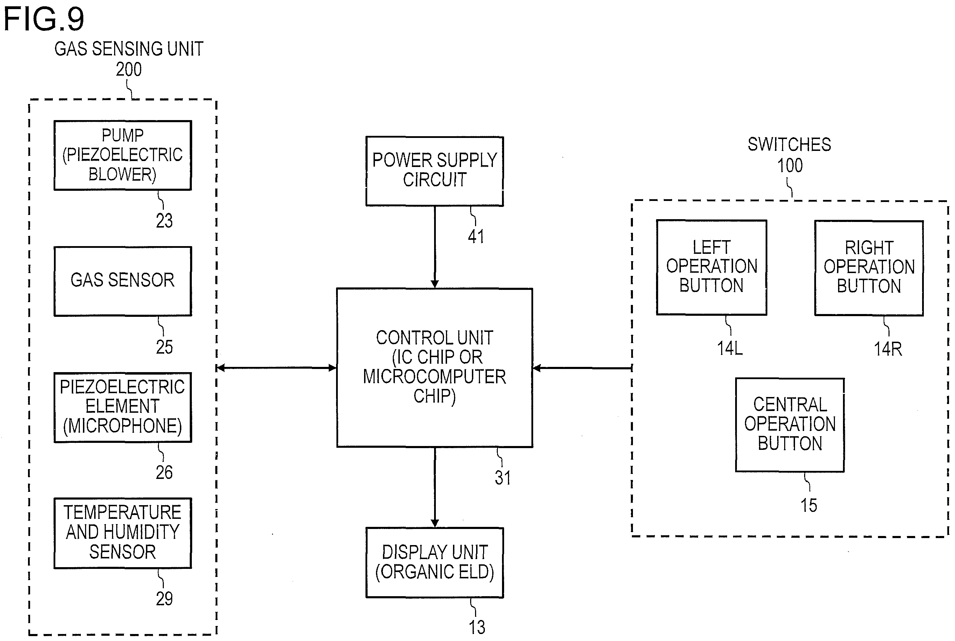

A control system of the breath component measurement apparatus 1 according to the present embodiment will be described below with reference to Fig. 9. As shown in Fig. 9, the control system of the breath component measurement apparatus 1 according to the present embodiment includes switches 100, a gas sensing unit 200, the display unit (for example, an organic ELD) 13, a power supply circuit 41, and a control unit (for example, an IC chip or a microcomputer chip) 31 which controls the constituent elements. The gas sensing unit 200 includes the pump (for example, a piezoelectric blower) 23, the gas sensor 25, and the piezoelectric element (for example, a microphone) 26 described earlier. The gas sensing unit 200 may include a temperature and humidity sensor 29 in addition to the constituent elements. The gas sensing unit 200 may include any other additional constituent element. The switches 100 include switches, such as the left operation button 14L, the right operation button 14R, and the central operation button 15 described earlier.

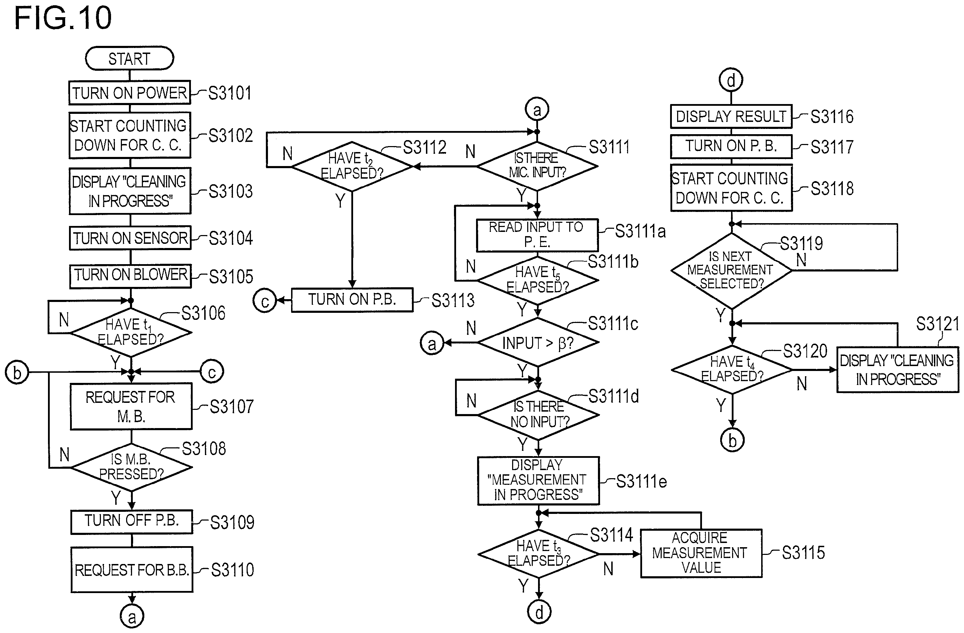

The operation of the control unit 31 will be described with reference to Fig. 10. If the power is turned on by a user operation (S3101), the control unit 31 starts counting down for a cleaning count time (S3102). The control unit 31 displays the message "CLEANING IN PROGRESS" on the display unit 13 (S3103). The control unit 31 controls the various sensors described earlier (for example, the gas sensor 25 and the temperature and humidity sensor 29) to be turned on (S3104). The control unit 31 controls the pump (piezoelectric blower) 23 to be turned on (S3105). The operations in steps S3102 to S3105 may be triggered by step S3101 and be simultaneously executed or may be executed in an order different from the above-described order.

The control unit 31 performs monitoring to see if t₁ (for example, 600) seconds as the cleaning count time have elapsed (S3106). If t₁ seconds have elapsed (Y in S3106), the control unit 31 displays a request for a measurement button (a request to a user for a press on the measurement button) on the display unit 13 (S3107). The measurement button is any operation button of the present apparatus, the display unit 13 (if the display unit 13 is touchable), or the like. If the user presses the measurement button (Y in S3108), the control unit 31 controls the pump (piezoelectric blower) 23 to be turned off and ends the cleaning processing (S3109). The control unit 31 then displays a request for breath blowing (a request to the user for breath blowing) on the display unit 13 (S3110). The control unit 31 performs monitoring to see if there is an input to the piezoelectric element 26 (a microphone input) (S3111). If t₂ (for example, 10) seconds have elapsed (Y in S3112) without sensing an input to the piezoelectric element 26 (a microphone input) (N in S3111), the control unit 31 judges that breath blowing is not performed, controls the pump (piezoelectric blower) 23 to be turned on, and restarts the cleaning processing (S3113). The process returns to step S3107. On the other hand, if an input to the piezoelectric element 26 (a microphone input) is sensed (Y in S3111) before a lapse of t₂ seconds (N in S3112), the control unit 31 judges that breath blowing is performed.

The control unit 31 then reads an input to the piezoelectric element 26 (a microphone input) for t₅ (for example, 0.2) seconds (S3111a and N in S3111b). After a lapse of t₅ seconds (Y in S3111b), the control unit 31 judges whether the read input to the piezoelectric element 26 exceeds β V (S3111c). Note that, if the piezoelectric element 26 is the microphone 26, the control unit 31 judges whether a microphone input count is not less than γ. If the input to the piezoelectric element 26 is judged to exceed β V (Y in S3111c; the microphone input count is not less than γ in a case where the piezoelectric element 26 is the microphone 26), it is judged that sufficient-intensity breath has flown in, and the control unit 31 waits until there is no longer any input to the piezoelectric element 26 (S3111 d). After the control unit 31 confirms that there is no longer any input to the piezoelectric element 26 (Y in S3111 d; after the control unit 31 confirms that there is no longer any microphone input in the case where the piezoelectric element 26 is the microphone 26), the process shifts to measurement value acquisition. For this reason, sufficient breath flows into the container 21. If the control unit 31 judges that the input to the piezoelectric element 26 is not more than β V (N in S3111 c), the process returns to S3111 for judging the presence of absence of a microphone input (N in S3111 c).

After the shift to measurement value acquisition (Y in S3111d), the control unit 31 produces the display "MEASUREMENT IN PROGRESS" or the like on the display unit 13 (S3111e; indicating to the user that measurement is in progress and prompting the user to wait). Until a lapse of t₃ (for example, 20) seconds (N in S3114), the control unit 31 continues to acquire a measurement value of a breath component accumulated in the container 21 by the gas sensor 25 (S3115).

After a lapse of t₃ seconds (Y in S3114), the control unit 31 displays an acquired measurement value or a value obtained by subjecting acquired measurement values to statistical processing on the display unit 13 (S3116). After that, the control unit 31 judges that the measurement is over, controls the pump (piezoelectric blower) 23 to be turned on, restarts the cleaning processing (S3117), and starts counting down for a cleaning count time (S3118). The control unit 31 then waits for an instruction for next measurement from the user (S3119). The instruction for next measurement from the user is given through a user operation of any operation button of the present apparatus or the display unit 13. If next measurement is selected (input) by the user (Y in S3119), the control unit 31 performs monitoring to see if t₄ (for example, 80, which may be set to be smaller than t₁ as the cleaning count time at the time of power-on) seconds which are set as the cleaning count time have elapsed (S3120). If t₄ seconds have not elapsed (N in S3120), the control unit 31 displays the message "CLEANING IN PROGRESS" on the display unit 13 (S3121). After a lapse of t₄ seconds (Y in S3120), the process returns to step S3107, and the control unit 31 displays a request for the measurement button (a request to the user for a press on the measurement button) on the display unit 13 (S3107).

### <Effects of Breath Component Measurement Apparatus 1>

In the breath component measurement apparatus 1 according to the present embodiment, the gas sensor 25 is arranged inside the breath residence case 2 that causes breath to reside (inside the container 21), which allows improvement in the accuracy of measuring a specific component in breath. Since the volume of the container 21 (or the breath residence case 2) is set to a capacity one-αth of a standard breath blowing amount per blowing, the concentration of the specific component in breath inside the container 21 is saturated, and a measurement result is unlikely to be affected by breath blowing intensity and a breath blowing time. This allows improvement in measurement accuracy.

Since the exhaust port 28 is configured to have a sufficiently small bore (for example, φ = 2.0 mm) with respect to the size of the container 21, breath inside the container 21 is unlikely to be exhausted, breath is likely to reside inside the container 21 during breath blowing, and the concentration of a specific component in breath is unlikely to change even after breath blowing. It is thus possible to improve the measurement accuracy.

The piezoelectric element 26 is provided close to the exhaust port 28, which allows sensing of the timing when breath blowing is started. Since the pump 23 is provided, gas inside the container 21 can be forcibly replaced with an ambient clean atmosphere, and cleaning of the gas sensor 25 under a clean atmosphere necessary before measurement can be performed.

The foregoing description of the embodiment of the invention has been presented for the purpose of illustration and description. It is not intended to be exhaustive and to limit the invention to the precise form disclosed. Modifications or variations are possible in light of the above teaching. The embodiment was chosen and described to provide the best illustration of the principles of the invention and its practical application, and to enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. A breath component measurement apparatus (1) comprising:
a breath residence case (2) that causes breath to reside; and
a gas sensor (25) that is provided inside the breath residence case (2).

2. The breath component measurement apparatus (1) according to Claim 1, wherein
the breath residence case (2) has a capacity one-αth of a capacity set in advance as a standard breath blowing amount per blowing, where α is a value set in advance.

3. The breath component measurement apparatus (1) according to Claim 2, wherein
α is set to be not less than 8.8

4. The breath component measurement apparatus (1) according to any one of Claims 1 to 3, wherein
the breath residence case (2) includes an intake port (225) and an exhaust port (28) and causes breath flowing in through the intake port (225) to reside.

5. The breath component measurement apparatus (1) according to Claim 4, further comprising:
a piezoelectric element (26) that senses exhaust from the exhaust port (28).

6. The breath component measurement apparatus (1) according to any one of Claims 1 to 5, further comprising:
a pump (23) that replaces gas inside the breath residence case (2).
